Europäisches Patentamt

European Patent Office

Office européen des brevets

(19)

(11) Veröffentlichungsnummer: **0 223 915 B1**

# EUROPÄISCHE PATENTSCHRIFT

(12)

(45) Veröffentlichungstag der Patentschrift :
16.01.91 Patentblatt 91/03

(51) Int. Cl.$^5$: **A61K 39/165**

(21) Anmeldenummer : 86110115.2

(22) Anmeldetag : 23.07.86

(54) **Lebendimpfstoff gegen Mumps und Verfahren zu dessen Herstellung.**

Die Akte enthält technische Angaben, die nach dem Eingang der Anmeldung eingereicht wurden und die nicht in dieser Patentschrift enthalten sind.

(30) Priorität : 26.11.85 CH 5062/85
15.01.86 CH 133/86

(43) Veröffentlichungstag der Anmeldung :
03.06.87 Patentblatt 87/23

(45) Bekanntmachung des Hinweises auf die
Patenterteilung :
16.01.91 Patentblatt 91/03

(84) Benannte Vertragsstaaten :
AT BE CH DE FR GB IT LI NL SE

(56) Entgegenhaltungen :
FR-A- 2 455 628
US-A- 3 961 046

(73) Patentinhaber : Schweizerisches Serum- und
Impfinstitut und Institut zur Erforschung der
Infektionskrankheiten
Rehhagstrasse 79
CH-3018 Bern (CH)

(72) Erfinder : Glück, Reinhard, Dr. phil. nat.
Blinzernfeldweg 8
CH-3098 Köniz (CH)
Erfinder : Germanier, René, Prof. Dr. phil. nat.
Mettlenhölzliweg 8
CH-3074 Muri bei Bern (CH)

(74) Vertreter : Zutter, Hans Johann Niklaus
EPROVA AG Forschungsinstitut Im
Laternenacker 5
CH-8200 Schaffhausen (CH)

## Beschreibung

Die Erfindung betrifft einen Impfstoff gegen Mumps, der an humane Zellen adaptiert ist, sowie das Verfahren zur Herstellung desselben aus virulenten Mumps-Viren, die aus akut an Mumps erkrankten Patienten stammen.

Mumps, Paroitis epidemica, ist eine noch sehr stark verbreitete Infektionskrankheit, die in vielen Fällen kaum Symptome verursacht, gewöhnlich zu einer Schwellung der Ohrspeicheldrüsen führt und im Kindesalter meistens gutartig verläuft.

Nach der Pubertät können gehäufte Komplikationen auftreten, beispielsweise Orchitis beim Mann oder Ovaritis bei der Frau, die zu Atrophien mitunter bis zur Sterilität führen können. Andere Komplikationen dieser Virusinfektion können das Zentralnervensystem betreffen und Enzephalitis, Enzephalomyelitis, Neuritis und Hirnhautentzündung verursachen.

Die grösste Krankheitshäufigkeit findet sich im Schulalter. In diesem Alter ist der Kontagionsindex am grössten. Nach einer Inkubationszeit von 18 bis 21 Tagen beginnt die akute febrile Phase. Die Ansteckungsfähigkeit beginnt 2 Tage vor der Drüsenschwellung und dauert bis zu deren Abschwellung. Eine durchgemachte Erkrankung oder Impfung mit attenuierten Mumpsviren verleiht lebenslängliche Immunität.

Im Hinblick auf mögliche Komplikationen von Mumps-Erkrankungen vor allem bei Erwachsenen ist die Verfügbarkeit eines optimal verträglichen Mumps-Impfstoffes, der zu einem hohen Antikörpertiter führt, unbedingt erforderlich. Die Schutzimpfung soll möglichst im 1. bis 3. Lebensjahr erfolgen.

Vielfach wird Mumps als eher harmlose Krankheit hingestellt und insbesondere Impfgegner weisen in der Öffentlichkeit oft unkritisch bloss auf mögliche Impfzwischenfälle hin. Dies kann dazu führen, dass in der Bevölkerung die Zahl von gegen Mumps geimpften Personen niedrig bleibt, womit automatisch die Gefahr an einer Mumpsinfektion mit ihren möglichen Komplikationen zu erkranken nach wie vor sehr hoch bleibt. Es ist daher ausserordentlich wichtig, einen Mumpsimpfstoff zur Verfügung zu stellen, der bezüglich Sicherheit und Wirksamkeit keine Wünsche offen lässt. Am sichersten und gleichzeitig wirksamsten sind vollständig attenuierte, an das Wachstum auf menschlichen Diploidzellen adaptierte, genetisch einheitliche Mumps-Virus-Populationen, die keine Fremdviren und keinerlei Reste von tierischem Eiweiss (Protein) enthalten.

In der Schweizer Patentschrift 475,355 ist ein Verfahren zur Züchtung von Viren, die einen Mumps-Impfstoff liefern, beschrieben. Das dort beschriebene Verfahren ist dadurch gekennzeichnet, dass der virulente Mumps-Virus mehreren Passagen in Hühnerembryogewebe-Kulturen unterworfen wird, bis er entsprechend abgeschwächt ist.

Dieses Verfahren weist den prinzipiellen Nachteil auf, dass der danach hergestellte Impfstoff an Hühnerembryogewebe-Kulturen adaptiert ist und noch Reste dieser Kulturen enthält. Die Fremdeiweisse aus der Impfflüssigkeit können unerwünschte Überempfindlichkeitsreaktionen hervorrufen. Schliesslich enthält der in Hühnerembryogewebe-Kulturen erzeugte Impfstoff zur Vermeidung bakterieller Kontamination Antibiotika wie beispielsweise Neomycin, was als unerwünscht angesehen wird.

In der amerikanischen Patentschrift 3'961'046 ist die Attenuierung von Mumps-Viren beschrieben mittels 3 konsekutiven Passagen durch primäre menschliche Amnionzell-rasen, gefolgt von einer Passage durch Hühnereier und 8 bis 18 konsekutive Passagen in primären Hühnerembryonal-gewebe. Der nach diesem Verfahren hergestellte Mumps-Impfstoff weist folgende schwerwiegende Nachteile auf : 1. Mögliche Kontamination mit Fremdviren ; 2. er besteht aus einer genetisch heterogenen Mumps-Virus-Population ; 3. Kontamination mit Fremdeiweiss (Protein), welches bei sensitiven Personen allergische Nebenreaktionen auslösen kann und 4. unbekannte Sicherheit und Immunogenität beim Menschen.

In der französischen Patentanmeldung (FR-A-) 2'455'628 wird die Herstellung von Mumps-Impfstoff enthaltend Viren vom Stamm Urabe beschrieben, durch Attenuie rung des Virus durch 1 bis 18 konsekutiven Passagen auf embryonaler Hühnereierchorioallantois-Membrane, menschlichen Diploidzellen oder Kulturen von Hühnerembryo-fibroblasten und Vermehrung der Viren auf einer oder allen oben genannten Zellsubstraten. Der nach diesem Verfahren erzeugte URABE-Impfstoff verursacht immer wieder gelegentlich Mumps-Meningitis. Seine Verwendung in Kanada ist daher nicht mehr erlaubt.

Der Erfindung liegt die Aufgabe zugrunde, einen Mumps-Impfstoff zu schaffen, der die beschriebenen Nachteile nicht aufweist, an das Wachstum auf menschlichen Diploidzellen adaptiert, optimal verträglich ist, keine lokale oder allgemeine Überempfindlichkeitsreaktionen stimuliert, auch bei Massenimpfungen nie neurologische Nebenwirkungen verursacht und ausser abgeschwächten harmlosen Mumps-Viren keine pharmakologisch aktiven Bestandteile aufweist

Die vorliegende Erfindung beschreibt eine Methode zur Herstellung eines neuen attenuierten Mumps-Virus-Stammes genannt "Rubini", welcher hoch attenuiert, an das Wachstum in menschlichen Diploid-Zellen adaptiert ist und keine neurologischen Nebenwirkungen (Enzephalomyelitis, Meningismus, Meningitis) verursacht

Es wurde gefunden, dass man Mumps-Viren, die von akut an Mumps erkrankten Patienten stammen, auf diploidem Humangewebe kultivieren, durch wiederholte Passagen auf diesem Gewebe und durch Passagen im Amnion- und Allantoissack von befruchteten Hühnereiern, gefolgt von weiteren Passagen in diploiden Humangewebe-Kulturen attenuieren und adaptieren und damit Ausgangsmaterial für einen Lebendimpfstoff gegen Mumps erzeugen kann

Zur Erzielung eines wirksamen Impfstoffes ist es zweckmässig, bei den Passagen auf verschiedenen Gewebekulturen, jeweils die Viren aus Passagen mit besonders hohem Titer für die Weiterverarbeitung auszuwählen

Nach dieser prinzipiellen Methode wurde auch der Mumps-Virusstamm Rubini, der aus dem Urin eines akut an Mumps erkrankten 8jährigen Knaben gewonnen wurde, durch Passagen in diploiden Humanzell-Kulturen attenuiert und adaptiert. Aus dem so gewonnen Material wurde ein für die Impfstoff-Produktion geeigneter SaatStamm vom Typus Rubini selektioniert.

Der so erhaltene selektionierte Mumps-Virus Stamm zeichnet sich durch eine sehr gute Vermehrungsrate in humanem Gewebe, rasche Ausbildung von besonders hohen Antikörpertitern beim Menschen sowie seine problemlose gute Verträglichkeit aus

Der damit gewonnene Human-Diploid-Cell(HDC)-Lebendimpfstoff gegen Mumps enthält keine tierische Fremdeiweisse und keine Antibiotika

Gegenstand der Erfindung ist demnach ein Lebendimpfstoff gegen Mumps, der dadurch gekennzeichnet ist, dass er Mumpsviren enthält, die aus dem Urin eines akut an Mumps erkrankten jungen Patienten gewonnen und durch

– zwei bis fünf aufeinander folgende Passagen in diploidem Humangewebe gefolgt von

– Passagen in der Amnion- und Allantoishöhle von befruchteten Hühnereiern gefolgt von

– Passagen in diploidem Humangewebe hoch attenuiert und an menschliches Gewebe adaptiert wurden und dass er keine Fremdviren, kein Fremdeiweiss und keine Antibiotika enthält.

Der Impfstoff ist weiter dadurch gekennzeichnet, dass er attenuierte Mumps-Viren vom Stamm Rubini, hinterlegt am 14.01.86 beim Institut Pasteur, C.N.C.M., Paris, O.-Nr. I-503, enthält

Das Verfahren zur Herstellung dieses Lebendimpfstoffes gegen Mumps ist dadurch gekennzeichnet, dass man

– virulente Mumpsviren aus dem Urin eines an Mumps erkrankten jungen Patienten durch Ultrazentrifugation isoliert,

– durch aufeinander folgende Passagen in diploidem Humangewebe vom Typus Wi-38 an dieses Gewebe adaptiert,

– anschliessend drei Passagen auf angebrüteten Hühnereiern bei 32 bis 35°C durchführt, wobei jeweils verschiedene Inokulations- und Erntemethoden angewandt werden, nämlich Inokulation in die und Ernte aus der Allantoishöhle, Inokulation in die und Ernte aus der Amnionhöhle sowie Inokulation in die Amnionhöhle und Ernte aus der Allantoishöhle resp. Amnionhöhle,

– anschliessend zehn weitere Passagen in angebrüteten Hühnereiern durchführt, jeweils durch Inokulation in die Amnionhöhle und Ernte aus der Allantoisflüssigkeit,

– gefolgt von vier aufeinander folgende schnellen Passagen in humandiploiden Zellen vom Typus MRC-5 bei 30°C,

– gefolgt von neun weiteren Passagen in MRC-5 Zellen bei 35°C,

– das so erhaltene attenuierte, an das Wachstum in menschlichem Gewebe adaptierte Saatvirus isoliert und

– in humandiploiden Rasen von MRC-5 Zellen vermehrt, danach erntet, reinigt, zum Impfstoff verarbeitet und diesen durch Gefriertrocknung stabilisiert

Das Verfahren ist weiter dadurch gekennzeichnet, dass man abgeschwächte, attenuierte und an Humangewebe-Kulturen adaptierte Mumps-Viren vom Stamm Rubini, hinterlegt am 14.01.86 beim Institut Pasteur, C.N.C.M., Paris, Ordnungsnummer I-503 kultiviert und zum Lebendimpfstoff verarbeitet.

Detaillierte Beschreibung der Herstellung von HDC-Mumpsimpfstoff

A Isolierung und Übertragung von virulentem Mumpsvirus

Die Isolierung des Virus aus Urin von einem an Mumps erkrankten Patienten geschieht durch Aufarbeitung in der Ultrazentrifuge (4 h, 25'000 U/min.). Die dabei gewonnene Mumps-Virus-haltige Fraktion (Bestimmung durch den Hämagglutinationstest) wird in diploiden Humanzellgewebe-Kulturen (z.B. Kulturen vom Typus Wi-38) und in angebrüteten Hühnereiern kultiviert. Das so isolierte Mumpsvirus wird anschliessend durch Ultrazentrifugation während 4 h bei 25'000 U/min. gereinigt und aufkonzentriert.

B Attenuierung, Abschwächung und Adaption

Zur Attenuierung und Adaption wurde die obige Mumps-Virus-Saat weiteren Passagen in befruchteten Hühnereiern und wiederum in humandiploiden Zellen unterworfen :

1. Mehrere Passagen im Amnion- oder Allantoissack resp. abwechselnd von angebrüteten Hühnereiern bei 30-35°C (z.B. 32°C).

2. Weitere Passagen in diploiden Humanzellgeweben vom Typus MRC-5 bei 30°C.

3. Neue Passagen in Zellgewebekulturen desselben Typus (MRC-5) bei 35°C.

C Herstellung des Impfstoffes

Das nach diesen aufeinander folgenden Passagen erhaltene Saatvirus wird auf humandiploiden Zellen wie z.B. auf MRC-5-Zellen vermehrt

Die Virussuspension wird geerntet und zum gebrauchsfertigen Impfstoff aufgearbeitet.

Dazu wird die erhaltene Virussuspension beispielsweise durch Zentrifugation oder Filtration geklärt, der Virusgehalt wird z.B. auf VERO-Zellen bestimmt, das Material wird durch Zusatz eines Zuckers wie Glucose, Lactose und/oder Saccharose stabilisiert und schliesslich lyophilisiert

Die Zahl der Passagen des Virus auf diploidem Humangewebe und auf befruchteten Hühnereiern können variiert werden. Ebenso kann die Temperatur während der Bebrütung und Kultivierung etwa zwischen 30 und 38°C variiert werden.

Zwei Hauptziele sind bei der Abschwächung (Attenuierung) und Kultivierung der Viren stets im Auge zu behalten :

     a) Abschwächung der krankmachenden Virulenz des Virus unter Erhalt von dessen antigenen Eigenschaften, d.h. der Fähigkeit die Bildung von Antikörpern zu induzieren

     b) Produktion einer attenuierten Viruskultur, die kein Fremdeiweiss - im vorliegenden Fall keine avianen Proteine - und keine Spuren von Antibiotika enthält.

Beispiel 1

Herstellung von HDC-Mumpsimpfstoff

A. Herstellung des Saatvirus

Aus Urin eines Mumpspatienten, erhalten am Ende der Virämie, wird das Mumpsvirus mittels Differential- und Ultrazentrifugation (4 h, 25'000 U/min.) gereinigt und aufkonzentriert. Die mumpsvirushaltige Fraktion wird mittels Hämagglutinationstest determiniert und in einem eiweiss-haltigen Medium (z.B. Basal-Medium-Eagle (BME) von Gibco + 0,4% humanem Serumalbumin (HSA)) aufgenommen und aliquotiert.

Ein Aliquot wird wie folgt weiterverarbeitet :
Ein frisch konfluenter humandiploider Zellrasen (z.B. aus Wi-38) wird mit der Mumpssuspension beimpft und nach Zugabe von Nährmedium (z.B. BME + 10% fötalem Rinderserum (FBS)) bei 37°C bebrütet. Nach mehreren Tagen wird der Zellrasen geerntet. Die erhaltene Ernte wird noch 1- bis 4mal auf humandiploiden Zellen passagiert

Anschliessend werden 3 Passagen auf SPF-Hühnereiern durchgeführt, wobei jeweils verschiedene Inokulations- und Ernte-Methoden angewandt werden :

     a) Inokulation in die und Ernte aus der Allantoishöhle

     b) Inokulation in die und Ernte aus der Amnionhöhle

     c) Inokulation in die Amnionhöhle und Ernte aus der Allantoishöhle resp. Amnionhöhle.

Die Eier werden zwischen 32 und 35°C bebrütet. Die nach diesen 3 Eipassagen mit den 8 verschiedenen Inokulations/Ernte Varianten erhaltenen Viren werden gepoolt.

Mit diesem Pool werden 10 weitere Eipassagen durchgeführt :
Inokulation in die Amnionhöhle, Ernte aus der Allantoisflüssigkeit. Die Temperatur wird bei 32°C gehalten. Der Mumpsantigentiter wird jeweils mittels Hämagglutinationstest bestimmt.

Anschliessend folgen 4 schnelle Passagen auf humandiploiden Zellen (MRC-5) bei 30°C (pro Passage ca 7 Tage). 9 weitere Passagen auf MRC-5 Zellen bei 35°C (pro Passage 10-20 Tage) ergeben das abgeschwächte und attenuierte Saatvirus.

B. Prüfung des Saatvirus

Das durch die Abschwächung erhaltene Saatvirus wird auf Mumpsidentität (im Neutralisationstest auf VERO-Zellen) und auf Viruskonzentration (Titer in VERO-Zellen : 5,6 $\log_{10}$ ID$_{50}$/ml) getestet. Das Saatvirus wurde als Mumpsvirus identifiziert und der Titer weist darauf hin, dass eine Produktion auf MRC-5-Zellen mit dem "Rubini"-Mumpsvirus möglich ist.

Zudem wurde das Saatvirus in folgenden Tests als mikrobiologisch rein befunden.

     – Test auf flüssigem Thioglycolat Medium bei 30-32°C : Frei von Bakterien

     – Tests auf flüssigem Soya Medium bei 20-25°C: Frei von Pilzen

     – Tests auf flüssigen und freien Medien : Frei von Mycoplasmen

     – Tests (in vivo) auf Meerschweinchen : Frei von Mycobact. tuberculosis

     – Tests (in vitro) auf flüssigen (nach Santon) und in festen (nach Löwenstein-Jensen) Medien : Frei von Myocab. tuberculosis

     – Tests auf Aviane Leukose : Frei von Retroviren

     – Tests auf Fremdviren (in vivo) auf Affen (Cynomolgous), Meerschweinchen, Mäusen, Baby-Mäusen (jünger als 24 h) und befruchteten Hühnereier : Fremdvirusfrei

     – Tests auf Fremdviren (in vitro) auf Primary monkey kidney und HDC (MRC-5) und Lung-18 : Fremdvirusfrei

C. Herstellung eines HDC-Mumpsimpfstoffes

Das Saatvirus wird auf humandiploiden Zellen (z.B. MRC-5 Zellen aus einer nach WHO-Requirements geprüften Zellbank) in zur Grossproduktion geeigneten Kulturflaschen (z.B. Rollerflaschen) zur Vermehrung gebracht. Nach einer Inkubation bei 35°C während 7-10 Tagen und nach mikroskopischer Beurteilung (Erkennen von Cytopathogenem Effekt) wird die Virussuspension in täglichen Abständen während ca 7 Tagen geerntet. Erhaltenes Virusbulkmaterial wird bis nach Erhalt der Prüfungsresultate bei –190°C (Gasphase von flüssigem Stickstoff) gelagert.

Das als in Ordnung befundene Virusbulkmaterial wird wieder aufgetaut und mit einem Filter der Porengrösse um 5 μm klarfiltriert.

Nach Verdünnung mit einem Stabilisator bestehend aus einer humanes Serum-Albumin enthaltenden Lösung von Lactose oder Lactose und Glucose wird der so erhaltene "Final Bulk" in 0,5 ml Portionen in 3 ml Fläschchen abgefüllt und im gefrorenen Zustand in Vakuo gefriergetrocknet.

D. Klinische Untersuchungen

Die bisher durchgeführten Untersuchungen weisen nach, dass die Impfung mit dem oben beschriebenen Impfstoff optimal vertragen wird. Es wurden weder Fieber noch irgendwelche lokale Reaktionen festgestellt. Die kritischen Organe wie beispielsweise Ohrspeicheldrüsen oder Hoden zeigten keinerlei Schwellungen, Entzündung oder irgendwelche schmerzhafte Reaktionen. Bis auf einen einzigen Fall zeigten alle Probanden eine positive Serokonversion. Der einzige Fall, welcher bei der Nachkontrolle keine Mumps-spezifischen Antikörper aufwies, hat früher auch negativ auf im Handel erhältliche Mumps-Impfstoffe reagiert.

In einer weiteren klinischen Untersuchung wurde der erhaltene HDC-Mumpsimpfstoff in Form einer kombinierten Mumps-Masern-Rötelnlebendvirus-Humandiploidzellvakzine (HDCV) in einem im Doppelblindverfahren durchgeführten Feldversuch gegen Mumps, Masern und Röteln getestet.

Insgesamt wurden 120 Kleinkinder im Alter von 15-20 Monaten in die Prüfung einbezogen. 60 Kinder erhielten den neuen Impfstoff, während eine Kontrollgruppe mit 60 Kinder mit dem vorbekannten Mumps-Masern-Röteln-Impfstoff M-M-R[R] II (Merck, Sharp & Dohme) geimpft wurden. 6 bis 8 Wochen nach der Impfung fanden sich in beiden Gruppen ohne Ausnahme hohe Serokonversionsraten (95-100%). Im multiplem $x^2$-Test ergab sich kein statistisch signifikanter Unterschied der immunogenen Wirksamkeit der beiden Impfstoffe (p >0,05). Berichte über irgendwelche Nebenwirkungen mit der HDCV trafen aus keiner der 12 am klinischen Versuch beteiligten ärztlichen Praxen ein.

Es wurde jedoch beobachtet, dass alle Komponenten des HDC-Impfstoffes einen hohen Attenuierungsgrad aufwiesen und dass der Impfstoff weder aviane Proteine, tierische Proteinextrakte noch Antibiotika enthält. Damit entfielen alle theoretischen und praktischen Kontraindikationen wegen entsprechenden Überempfindlichkeiten. Es wurden in keinem Fall Nebenwirkungen beobachtet.

Beispiel 2

Herstellung von HDC-Mumpsimpfstoff
A) Isolierung der Mumsviren wie im Beispiel 1 beschrieben
B) Vermehrung der Viren auf diploidem Humanzellgewebe.
C) Attenuierung durch 10 Passagen in diploidem Humanzellgewebe bei 30-35°C, 6 Passagen abwechselnd im Amnion- und Allantoissack von ausgebrüteten Hühnereiern bei 35-38°C und schliesslich 6 schnelle und 10 normale Passagen in diploidem Humanzellgewebe MRC-5 bei 30-35° C.
D) Vermehrung (Produktion) des nach A/B/C erhaltenen Saatvirus auf humandiploiden Zellen.
E) Ernten der Viren, Trennung vom Kulturmedium, Reinigung, Konzentrierung, Prüfung, Stabilisierung und Lyophilisierung analog wie im Beispiel 1 beschrieben.

**Ansprüche**

1. Lebendimpfstoff gegen Mumps, dadurch gekennzeichnet, dass er Mumpsviren enthält, die aus dem Urin eines akut an Mumps erkrankten jungen Patienten gewonnen und durch
– zwei bis fünf aufeinander folgenden Passagen in diploidem Humangewebe, gefolgt von
– Passagen in der Amnion- und Allantoishöhle von befruchteten Hühnereiern, gefolgt von
– Passagen in diploidem Humangewebe hoch attenuiert und an menschliches Gewebe adaptiert wurden und dass er keine Fremdviren, kein Fremdeiweiss und keine Antibiotika enthält.

2. Impfstoff nach Patentanspruch 1, dadurch gekennzeichnet, dass er attenuierte Mumps-Viren vom Stamm Rubini, hinterlegt am 14.01.1986 beim Institut Pasteur, C.N.C.M., Paris, Ordnungsnummer I-503, enthält.

3. Verfahren zur Herstellung eines Lebendimpfstoffes gegen Mumps, dadurch gekennzeichnet, dass man
– virulente Mumpsviren aus dem Urin eines an Mumps erkrankten jungen Patienten durch Ultrazentrifugation isoliert,
– durch aufeinander folgende Passagen in diploidem Humangewebe vom Typus Wi-38 an dieses adaptiert,
– anschliessend drei Passagen auf angebrüteten Hühnereiern bei 32 bis 35°C durchführt, wobei jeweils verschiedene Inokulations- und Erntemethoden angewandt werden, nämlich Inokulation in die und Ernte aus der Amnionhöhle sowie Inokulation in die Amnionhöhle und Ernte aus der Allantoishöhle resp. Amnionhöhle,
– anschliessend zehn weitere Passagen in angebrüteten Hühnereiern durchführt, jeweils durch Inokulation in die Amnionhöhle und Ernte aus der Allantoisflüssigkeit,

– gefolgt von vier aufeinander folgenden schnellen Passagen in humandiploiden Zellen vom Typus MRC-5 bei 30°C,

– gefolgt von neun weiteren Passagen in MRC-5-Zellen bei 35°C,

– das so erhaltene attenuierte, an das Wachstum in menschlichem Gewebe adaptierte Saatvirus isoliert und

– in humandiploiden Rasen von MRC-5-Zellen vermehrt, danach erntet, reinigt, zum Impfstoff verarbeitet und diesen durch Gefriertrocknung stabilisiert.

4. Verfahren nach Patentanspruch 3, dadurch gekennzeichnet, dass man abgeschwächte, attenuierte und an Humangewebe-Kulturen adaptierte Mumps-Viren vom Stamm Rubini, hinterlegt am 14.01.1986 beim Institut Pasteur, C.N.C.M., Paris, Ordnungsnummer I-503, kultiviert und zum Lebendimpfstoff verarbeitet.

## Claims

1. Live vaccine against mumps, characterized in that it contains mumps viruses which have been obtained from the urine of a young patient with acute mumps and have been highly attenuated and adapted to human tissue by

– two to five consecutive passages in diploid human tissue, followed by

– passages in the amniotic and allantoic cavity of fertilized chicken eggs, followed by

– passages in diploid human tissue and in that it contains no foreign viruses, no foreign protein and no antibiotics.

2. Vaccine according to patent Claim 1, characterized in that it contains attenuated mumps viruses of the Rubini strain, deposited on 14.01.1986 at the Institut Pasteur, C.N.C.M., Paris, file number I-503.

3. Process for the preparation of a live vaccine against mumps, characterized in that

– virulent mumps viruses are isolated by ultracentrifugation from the urine of a young patient with mumps,

– adapted to diploid human tissue of the type Wi-38 by consecutive passages therein,

– subsequently three passages are carried out on partly incubated chicken eggs at 32 to 35°C, using different inoculation and harvesting methods in each case, namely inoculation into and harvesting from the amniotic cavity, and inoculation into the amniotic cavity and harvesting from the allantoic cavity and amniotic cavity respectively,

– subsequently ten further passages are carried out in partly incubated chicken eggs, in each case by inoculation into the amniotic cavity and harvesting from the allantoic fluid,

– followed by four consecutive rapid passages in human diploid cells of the type MRC-5 at 30°C,

– followed by nine further passages in MRC-5 cells at 35°C,

– the attenuated seed virus which is obtained in this way and is adapted to growth in human tissue is isolated and

– grown in human diploid lawns of MRC-5 cells, then harvested, purified, processed to the vaccine, and the latter is stabilized by freeze drying.

4. Process according to patent Claim 3, characterized in that weakened and attenuated mumps viruses of the Rubini strain, deposited on 14.01.1986 at the Institut Pasteur, C.N.C.M., Paris, file number I-503, which have been adapted to human tissue cultures are cultivated and processed to the live vaccine.

## Revendications

1. Vaccin vivant contre les oreillons, caractérisé en ce qu'il contient des virus des oreillons, qui sont obtenus à partir de l'urine d'un jeune patient atteint d'oreillons en phase aiguë et ont été hautement atténués par

– deux à cinq passages succesifs dans du tissu diploïde humain, suivis de

– passages dans la cavité amniotique et allantoïde d'oeufs de poule fécondés, suivis de

– passages dans du tissu diploïde humain et adaptés au tissu humain, et caractérisé en ce qu'il ne contient ni virus étrangers, ni protéines étrangères ni antibiotiques.

2. Vaccin selon la revendication 1, caractérisé en ce qu'il contient des virus atténués des oreillons de la souche Rubini, déposée le 14.01.1986 à l'Institut Pasteur, C.N.C.M., sous le n° I-503.

3. Procédé de préparation d'un vaccin vivant contre les oreillons, caractérisé en ce

– qu'on isole des virus virulents des oreillons à partir de l'urine d'un jeune patient atteint d'oreillons par ultracentrifugation,

– que, par des passages successifs dans du tissu dipoloïde humain du type Wi-38, on l'adapte à ce dernier,

– qu'on effectue ensuite trois passages sur des oeufs de poule incubés à 32-35°C, en utilisant chaque fois des méthodes d'inoculation et de récolte différentes, à savoir, inoculation dans la cavité amniotique et récolte à partir de cette dernière ainsi qu'inoculation dans la cavité amniotique et récolte resp. à partir de la cavité allantoïde et amniotique,

– qu'on effectue ensuite dix autres passages dans des oeufs de poule incubés, chaque fois par inoculation dans la cavité amniotique et récolte à partir du liquide allantoïde,

– suivis de quatre passages rapides successifs dans des cellules diploïdes humaines du type MRC-5 à 30°C,

    – suivis de neuf autres passages dans des cellules MRC-5 à 35°C,

    – qu'on isole le virus d'ensemencement obtenu atténué, adapté à la croissance dans le tissu humain et

    – qu'on le multiplie dans des cultures de cellules diploïdes humaines MRC-5, qu'on le récolte, le purifie, qu'on le transforme en vaccin et qu'on stabilise ce dernier par lyophilisation.

    4. Procédé selon la revendication 3, caractérisé en ce qu'on cultive et transforme en vaccin vivant des virus des oreillons affaiblis, atténués et adaptés aux cultures de tissu humain, de la souche Rubini, déposée le 14.01.1986 à l'Institut Pasteur, C.N.C.M., Paris, n° I-503.